# EUROPEAN PATENT APPLICATION

(11) **EP 4 342 907 A1**
(43) Date of publication of application: **27.03.2024**
(21) Application number: 22196890.2
(22) Date of filing: 21.09.2022
(51) Int. Cl.: C07K 14/725, A61K 38/00, A61P 35/00, C07K 14/705, C07K 14/715, C07K 16/00, C12N 5/0783, C07K 19/00

(54) **SWITCHABLE CHIMERIC ANTIGEN RECEPTORS AND THEIR USE**

(71) Applicant: AvenCell Europe GmbH, 01307 Dresden (DE)
(72) Inventor: ZIMMERMANN, Luise, 01307 Dresden (DE); LOFF, Simon, 01307 Dresden (DE); SPEHR, Johannes, 01307 Dresden (DE); EHNINGER, Armin, 01307 Dresden (DE); CARTELLIERI, Marc, 01307 Dresden (DE)
(74) Representative: Kailuweit & Uhlemann Patentanwälte Partnerschaft mbB

(57) **Abstract**

The present invention relates to a switchable chimeric antigen receptor (CAR) comprising a tag or tag-binding domain, which is capable of binding a tag-binding domain or tag of a targeting module, wherein the targeting module comprises a target cell-binding domain. The invention also relates to a nucleic acid, vector or cell comprising a nucleotide sequence encoding the switchable CAR, a pharmaceutical composition comprising a nucleic acid, vector or cell comprising a nucleotide sequence encoding the switchable CAR, a kit and the use as a medicament, in particular for use in the treatment of cancer, infectious disease or autoimmune disease.

## Description

The present invention relates to a switchable chimeric antigen receptor (CAR) comprising a tag or tag-binding domain, which is capable of binding a tag-binding domain or tag of a targeting module, wherein the targeting module comprises a target cell-binding domain. The invention also relates to a nucleic acid, vector or cell comprising a nucleotide sequence encoding the switchable CAR, a pharmaceutical composition comprising a nucleic acid, vector or cell comprising a nucleotide sequence encoding the switchable CAR, a kit and the use as a medicament, in particular for use in the treatment of cancer, infectious disease or autoimmune disease.

Chimeric antigen receptors (CARs) are artificial receptors consisting of a binding moiety, which provides the antigen-specificity, e.g. a single chain variable fragment (scFv) recognizing a surface antigen of a tumor cell, a transmembrane domain, and one or several signaling chains derived from immune receptors, e.g. CD3ζ, that activates an immune cell (Cartellieri *et al.* 2010).

The first generation CAR comprising one signaling chain was modified by adding an intracellular signaling domain of CD28, which is a co-stimulatory molecule of a T cell, to increase the activation of immune cells (second generation CAR). A third generation CAR was developed by tandemly linking an intracellular signaling domain derived from CD137 (4-1BB) or CD134 (OX40), both which are tumor necrosis factor (TNF) receptor superfamily members, to a second generation CAR. However, not all intracellular signaling domains derived from every T cell signal transducing protein sufficiently stimulate a T cell to damage and/or kill a target tumor cell. Therefore, finding intracellular signaling domains of signal transducing proteins that are effective when linked to a CAR is desirable.

WO 2016/127257 A1 describes a CAR which specifically binds to a target antigen and imparts a cytotoxic activity against a target cell expressing the target antigen comprising an extracellular domain capable of binding to a predetermined antigen, a transmembrane domain and an intracellular segment comprising a) one or more intracellular signaling domains selected from a cytoplasmic domain of an interleukin receptor chain and/or a cytoplasmic co-stimulatory domain and b) a CD3ζ intracellular signaling domain comprising an exogenous STAT3 association motif, wherein the intracellular segment comprises an endogenous or exogenous JAK-binding motif and STAT5 association motif.

WO 2018/140725 A1 discloses the use of immune effector cells (e.g., T cells, NK cells) engineered to express a CAR polypeptide comprising an antigen binding domain, a transmembrane domain, and an intracellular domain that comprises a mutant CD28 costimulatory domain (RSKRSRLLHSDX₁MX₂MTPRRPGPTRKHYQPYAPPRDFAAYRS, wherein X₁ is any amino acid, X₂ is selected from R, C, E, G, H, I, L, M, F, S, T, W, Y, or V) that binds to a tumor antigen to treat cancer associated with expression of said tumor antigen.

WO 2013/126733 A1 describes a CAR comprising an antigen binding domain, a transmembrane domain and an ICOS intracellular signaling domain, preferably further comprising a CD3zeta signaling domain and/or a costimulatory signaling region comprising the intracellular domain of a costimulatory molecule selected from the group consisting of CD27, CD28, 41BB, OX40, CD30, CD40, PD-1, lymphocyte function-associated antigen-1 (LFA-1), CD2, CD7, LIGHT, NKGZC, B7-H3, a ligand that specifically binds with CD83, and any combination thereof.

Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al.* 2010). Moreover, as CAR T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CAR T cells to very few indications. In fact, examples of clinical effectiveness have mostly been seen with CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri *et al.* 2016). Antigen-specificity is provided by soluble adapter molecules, which consist of an antigen-binding domain fused to the tag recognized by the UniCAR. Cartellieri *et al.* describe the treatment of CD33- and/or CD123-positive acute myeloid leukemia cells *in vitro* and *in vivo.*

Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell binding domain (EP 3 581 200 A1).

Moreover, switchable CAR T approaches like UniCAR or RevCAR provide the possibility to rest CAR T cells in-between cycles of activation and stimulation by pausing administration of the soluble targeting module molecule. This is expected to prevent the exhaustion observed upon continuous stimulation of conventional CAR T cells thereby improving persistence (Weber *et al.* 2021).

The object of the present invention is therefore to provide a switchable chimeric antigen receptor sufficiently stimulating a T cell to damage and/or kill target cells.

According to the invention, the object is solved by the switchable chimeric antigen receptor, the nucleic acid, vector or cell comprising a nucleotide sequence encoding the switchable CAR, the pharmaceutical composition, and the kit according to the independent claims. Advantageous embodiments of the invention are indicated in the dependent claims.

A first aspect of the invention is a switchable chimeric antigen receptor (CAR) comprising
a. a tag or tag-binding domain,
b. an extracellular hinge and transmembrane domain comprising an extracellular hinge and a transmembrane domain of CD8α, CD28, ICOS (CD278) or mutants and combinations thereof,
c. an intracellular signaling domain that comprises at least two signal transduction domains independently selected from the group comprising a cytoplasmic region of CD3, preferably CD3ζ; CD28, 4-1BB (CD137), ICOS (CD278), IL-2, preferably IL-2Rβ; IL-7, preferably IL-7Rα; IL-15 or IL-21 and mutants thereof.

Advantageously, the switchable CAR according to the invention used in combination with a targeting module actively targets target cells, e.g. tumor cells, and is capable of inducing a significant anti-tumor response, wherein the anti-tumor response of the switchable chimeric antigen receptor is only induced in the presence of the targeting module. The effect can be reversibly interrupted by withholding the administration of the targeting module. Further advantageously, the pharmacokinetic and pharmacodynamic half-life of the targeting module is short, providing a rapid and reversible switch-off mechanism of the mediated immune response. Advantageously, the additional co-stimulus resulting from at least two signal transduction domains results in a long-term functionality or efficiency, respectively, of the switchable CAR T cell, and/or a lower exhaustion.

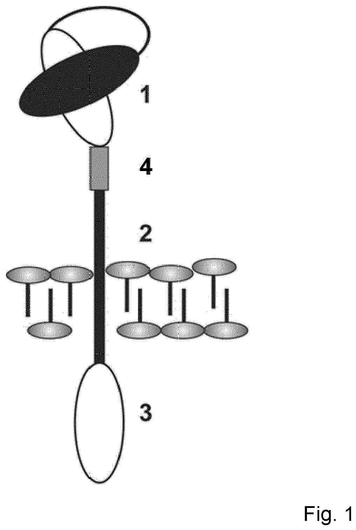

As used herein, the term "switchable chimeric antigen receptor" refers to an artificial chimeric fusion protein, in particular a receptor comprising a tag or a tag-binding domain, an extracellular hinge and a transmembrane domain and an intracellular signaling domain (Fig. 1). The domains can be derived from different sources and therefore, the receptor is called chimeric. Advantageously, the receptor can bind with the tag or tag-binding domain to the tag-binding domain or tag of different targeting modules, which in turn bind to an antigen on a target cell. Thus, the tag or tag-binding domain serves as target cell binding domain.

As used herein, the term "domain" refers to a part of a protein sequence, which can exist and function independently from the rest of the protein.

As used herein, the term "targeting module" refers to a molecule, preferably a polypeptide or protein with at least two different domains, wherein each domain is specific for a target or a uniform group of targets, respectively, wherein at least one domain is specific for a target cell, e.g. a CD123-binding domain; and one domain is specific for a switchable chimeric antigen receptor, in particular the tag or tag-binding domain.

As used herein, the term "target cell-binding domain" refers to a peptide, protein, or low molecular weight organic ligand, which specifically binds a protein or protein complex (antigen) on the surface of a target cell, preferably a cancer cell, T cell, infected cell, pathogens or parasites.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the terms "peptide", "polypeptide" and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

As used herein, the term "mutants" refers to peptides or proteins having at least 90 % sequence identity to the named antibodies, antibody fragments, proteins or peptides, preferably at least 95 % sequence identity. Advantageously, the mutants are capable of having one or more activities of the named domains, antibodies, antibody fragments, peptides or proteins.

In embodiments, the mutant comprises a point mutation. As used herein, a "point mutation" is a mutation, wherein a single nucleotide base is changed, inserted or deleted from nucleotide sequence. In embodiments, mutants are truncated variants of peptides or proteins. As used herein, the term "truncated versions" refers to shortened peptides or proteins having at least 90 % sequence identity to the named peptides or proteins, preferably at least 95 % sequence identity, more preferably having a chain length of at least 90 % and a sequence identity of 100 %, most preferably a chain length of at least 95 % and a sequence identity of 100 %, resulting from a mutation in the nucleotide sequence coding for the peptide or protein. Advantageously, the truncated version has at least 80 %, preferably of at least 90 %, more preferably of at least 95 %; of the activity of the named peptide or protein.

In embodiments, the intracellular signaling domain comprises at least two signal transduction domains independently selected from the group comprising a cytoplasmic region of CD3, a CD3 mutant; CD28, a CD28 mutant, 4-1BB (CD137), a 4-1BB mutant, ICOS (CD278), a ICOS mutant, IL-2, an IL-2 mutant, IL-7, an IL-7 mutant, IL-15, an IL-15 mutant, IL-21 or an IL-21 mutant, wherein preferably the CD3 mutant, CD28 mutant, 4-1BB mutant and/or ICOS mutant comprise one or two point mutations and/or wherein the IL-15 mutant and/or IL-21 mutant are truncated variants. Preferably, the IL-15 mutant and/or IL-21 mutant are truncated variants, wherein inhibitory areas are truncated.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g. FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 1, SEQ ID No. 2 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 3 or SEQ ID No. 4 or mutants thereof; or a short linear peptide sequence from a human protein, preferably from a human nuclear protein, more preferably from the human La protein, even more preferably according to SEQ ID No. 5, SEQ ID No. 6, SEQ ID No. 7 or mutants thereof.

As used herein, the term "nuclear protein" refers to a protein found in the cell nucleus. Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the human protein is a human Alpha-fetoprotein and human nuclear proteins, more preferably from the human La protein.

In embodiments, the His-tag is an amino acid sequence consisting of histidine residues, preferably in the range of six to fourteen histidine residues.

In preferred embodiments, the peptide epitope tag is a myc-tag, a His-tag, a short linear peptide sequence from yeast transcription factor GCN4, preferably according to SEQ ID No. 1 or SEQ ID No. 2; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 3 or SEQ ID No. 4; or a short linear peptide sequence from a human nuclear protein, preferably from the human La protein according to SEQ ID No. 5, SEQ ID No. 6 or SEQ ID No. 7.

Preferably, the peptide epitope tag from the human La protein is the human La epitope E5B9 according to SEQ ID No. 5 or E7B6 according to SEQ ID No. 6 or SEQ ID No. 7, most preferably the human La epitope E5B9 according to SEQ ID No. 5 or E7B6 according to SEQ ID No. 7.

In embodiments, the tag-binding domain is an antibody or antigen-binding fragment, a protein or a peptide.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment or antigen-binding fragment" refers to a protein comprising at least the V_{L} or V_{H} of an antibody. In an embodiment, antibody fragments are selected from single-chain variable fragments (scFv), single-chain antibodies, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

Preferably, V_{H} and V_{L} are connected via a glycine-serine linker with the structure (GₓS_{y}) with x and y selected from 1 to 10, preferably 1 to 5. Mostly preferred are 1 to 10 repeats of the sequence G₄S₁ (SEQ ID No. 13). Moreover, linkers are preferred that are constituted of a peptide sequence that can increase the protease resistance of the antibody derivatives. As used herein, the term "derivative" refers to a molecule having a high degree of structural identity to the molecule, preferably the same scaffold, wherein at least one atom, group of atoms, functional group or substructure is replaced with another atom, group of atoms functional group or substructure, e.g. a hydroxy group. Advantageously, the derivative is capable of having one or more activities of the named molecule.

In embodiments, the linker is SEQ ID No. 14 or SEQ ID No. 15.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on *in silico* peptide-MHC affinity prediction.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, the antibody or antibody fragment is a polyclonal, a monoclonal or a chimeric antibody, wherein an antigen-binding region of a non-human antibody is transferred into the framework of a human antibody by recombinant DNA techniques including *in silico* design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the tag-binding domain is an antibody, antigen-binding fragment, a protein or a peptide binding to a myc-tag, a His-tag, a short linear peptide sequence from yeast transcription factor GCN4, a leucine zipper sequence or a short linear peptide sequence from a human protein, preferably form a human nuclear protein, most preferably from the human La protein.

In preferred embodiments, the tag-binding domain is an antibody or an antibody fragment

In embodiments, the tag-binding domain binds to a tag from the human nuclear La protein; preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 8), wherein X₂₄, X₃₅, X₆₁ and X₁₀₁ are independently from each other selected from a proteinogenic alpha-amino acid residue;
or a sequence having at least 90 % sequence identity, preferably at least 95 % sequence identity; to one of the sequences SEQ ID No. 10 or SEQ ID No. 12.

In embodiments, the tag-binding domain constitutes an anti-La epitope scFv.

In some embodiments, X₂₄, X₃₅, X₆₁ and X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment comprising a V_{H} sequence having at least 90 % sequence identity, preferably at least 95 % sequence identity; to one of the sequences SEQ ID No. 9 or SEQ ID No. 11.

In embodiments, the tag-binding domain comprises a sequence having each at least 90 % sequence identity, preferably at least 95 % sequence identity; to the sequences according to SEQ ID NO. 9 (V_{H}) and SEQ ID NO. 10 (V_{L}) or the sequences according to SEQ ID NO. 11 (V_{H}) and SEQ ID NO. 12 (V_{L}).

In embodiments, the tag-binding domain constitutes the anti-La 5B9 scFv according to SEQ ID NO. 9 (V_{H}) and SEQ ID NO. 10 (V_{L}) or the anti-La 7B6 scFv according to SEQ ID NO. 11 (V_{H}) and SEQ ID NO. 12 (V_{L}).

In embodiments, the tag-binding domain is an antibody or antigen-binding fragment binding to a human La epitope E5B9 or E7B6 comprising a V_{L}-linker-V_{H} structure, wherein the V_{L} region of the tag-binding domain comprises a sequence with at least 95 % identity, preferably 99 % identity, with the sequence according to SEQ ID No. 10 or SEQ ID No. 12 and/or the V_{H} region of the tag-binding domain comprises a sequence with at least 95 % identity, preferably 99 % identity, with the sequence according to SEQ ID No. 9 or SEQ ID No. 11.

As used herein, the term "extracellular hinge and transmembrane domain" refers to a flexible peptide sequence connected to the tag or tag-binding domain, which anchors the switchable CAR into the cell membrane of the cell and protrudes from the surface of the cell for optimal binding to its particular targeting module.

According to the invention, the extracellular hinge and transmembrane domain comprise an extracellular hinge and a transmembrane domain of CD8α, CD28, ICOS (CD278) or mutants and combinations thereof. As used herein, the term "combinations thereof" refers to combinations of the different extracellular hinge and transmembrane domains.

In embodiments, combinations of the extracellular hinge and transmembrane domain are CD8α extracellular hinge and transmembrane domain, CD28 extracellular hinge and transmembrane domain, CD28 extracellular hinge domain combined with a CD8α or ICOS transmembrane domain, preferably a CD28 extracellular hinge and transmembrane domain or a CD28 extracellular hinge domain combined with an ICOS transmembrane domain.

In preferred embodiments, the extracellular hinge and transmembrane domain comprises an extracellular hinge domain of CD28, preferably a mutant of an extracellular hinge domain of CD28, more preferably a mutant of an extracellular hinge domain of CD28 comprising two point mutations in the B7 binding site. Advantageously, the mutation of the B7 binding site results in an abrogated ligand binding. Preferably, the extracellular hinge and transmembrane domain comprises a mutant of extracellular hinge domain of CD28 according to SEQ ID No. 16.

In embodiments, the extracellular hinge and transmembrane domain comprises SEQ ID No. 16 and SEQ ID No. 17 or SEQ ID No. 18.

In embodiments, the intracellular signaling domain comprises two or three signal transduction domains selected from the group comprising CD28, 4-1BB, ICOS, CD3ζ, IL-7Rα and mutants thereof.

In embodiments, the intracellular signaling domain comprises at least a cytoplasmic region of CD28 or mutants thereof, preferably according to SEQ ID No. 19 or SEQ ID No. 20.

In embodiments, the intracellular signaling domain comprises at least a cytoplasmic region of CD3ζ or mutants thereof, preferably according to one of the sequences SEQ ID No. 21 to SEQ ID No. 23.

In embodiments, the intracellular signaling domain comprises at least a cytoplasmic region of CD28 or mutants thereof, preferably according to SEQ ID No. 19 or SEQ ID No. 20, and a cytoplasmic region of CD3ζ or mutants thereof, preferably according to one of the sequences SEQ ID No. 21 to SEQ ID No. 23.

In further embodiments, the intracellular signaling domain comprises at least a cytoplasmic region of ICOS or mutants thereof, preferably according to SEQ ID No. 24.

In embodiments, the intracellular signaling domain comprises at least a cytoplasmic region of ICOS or mutants thereof, preferably according to SEQ ID No. 24, and a cytoplasmic region of CD3ζ or mutants thereof, preferably according to one of the sequences SEQ ID No. 21 to SEQ ID No. 23.

Preferably, the switchable chimeric antigen receptor comprises a CD28 transmembrane domain and as intracellular signaling domain at least one cytoplasmic region of CD28 or mutants thereof, preferably according to SEQ ID No. 19 or SEQ ID No. 20, or an ICOS transmembrane domain and as intracellular signaling domain at least one cytoplasmic region of ICOS or mutants thereof, preferably according to SEQ ID No. 24.

In embodiments, the intracellular signaling domain comprises further a cytoplasmic region of a cytoplasmic region of 4-1BB, preferably according to SEQ ID No. 25, IL-2Rβ, IL-7Rα and mutants thereof, preferably according to one of the sequences SEQ ID No. 25 to 27, more preferably a cytoplasmic region of 4-1BB, IL-7Rα and mutants thereof, preferably according to SEQ ID No. 25 or 26, most preferably a cytoplasmic region of 4-1BB and mutants thereof.

In embodiments, the switchable CAR is a universal chimeric antigen receptor comprising as element a.) a tag-binding domain.

In embodiments, the switchable CAR is a reversible chimeric antigen receptor comprising as element a.) a tag. Advantageously, cells comprising a reversible universal chimeric antigen cell surface receptor are less exhausted after stimulation thereby improving persistence.

In further embodiments, the switchable chimeric antigen receptor comprises a further domain, wherein the further domain is a short peptide linker in the extracellular portion of the receptor that may serve to detect the chimeric antigen receptor on the cell surface or stimulate the chimeric antigen receptor T cell.

In preferred embodiments, the further domain forms a linear epitope for a monoclonal antibody (mab) specifically binding to the further domain. In some embodiments, the further domain comprises at least one linear epitope, preferably E7B6 according to SEQ ID No. 6 or SEQ ID No. 7.

In some embodiments, the further domain is located in between the tag-binding domain or the tag and the extracellular hinge domain or an integral part of the extracellular hinge domain.

Advantageously, the switchable CAR engrafted cells with the further domain can be specifically stimulated to proliferate preferentially and persist longer compared to non-engrafted cells either *in vitro* or *in vivo.* Further advantageously, the further domain may also be used to purify switchable CAR engrafted cells from mixed cell populations or to dampen switchable CAR engrafted cell-mediated immune response and to eliminate switchable CAR engrafted cells *in vivo.*

In further embodiments, the switchable CAR comprises a signal peptide. Advantageously, the signal peptide allows for expression on the cell surface of an effector cell. In embodiments, the signal peptide is located at the N-terminus of the switchable CAR sequence in front of the tag-binding domain or the tag. In some embodiments, the signal peptide targets proteins to the secretory pathway either co-translationally or post-translationally and is selected from leader peptides from proteins like CD28, CD8α, IL-2, lysozyme C or the heavy or light chains of antibodies of human origin to avoid immunogenic reactions.

In embodiments, the tag-binding domain or tag is present at the amino-terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the switchable CAR comprises a sequence according to SEQ ID No. 28 to SEQ ID No. 45.

In embodiments, the switchable CAR is a reversible CAR and comprises a sequence according to SEQ ID No. 28 to SEQ ID No. 36, preferably according to SEQ ID No. 28, SEQ ID No. 29, SEQ ID No. 30, SEQ ID No. 33 or SEQ ID No. 35, more preferably according to SEQ ID No. 28, SEQ ID No. 29, SEQ ID No. 30 or SEQ ID No. 35.

In further embodiments, the switchable CAR is a UniCAR and comprises a sequence according to SEQ ID No. 37 to SEQ ID No. 45, preferably according to, SEQ ID No. 37, SEQ ID No. 38, SEQ ID No. 39 SEQ ID No. 42 or SEQ ID No. 44, more preferably according to SEQ ID No. 37, SEQ ID No. 38, SEQ ID No. 39 or SEQ ID No. 44.

Another aspect of the invention is a nucleic acid, vector or cell comprising a nucleotide sequence encoding the switchable CAR according to the invention.

In embodiments, the vector is selected from the group comprising a DNA vector, an RNA vector, a plasmid, a lentiviral vector, retroviral vector, adenoviral vector and an adeno-associated viral vector.

In embodiments, the vector further comprises a promoter, wherein the promoter is selected from the group comprising an EF-1 promoter, a CMV IE gene promoter, an EF-1α promoter, an ubiquitin C promoter, or a phosphoglycerate kinase (PGK) promoter.

In embodiments, the cell comprises an exogenous nucleotide sequence encoding the switchable CAR that is expressed on the surface of the cell.

In embodiments, the cell is an immune effector cell selected from the group comprising a T cell, preferably a cytotoxic T lymphocyte (CTL) or regulatory T cell (T_{reg}); Natural Killer (NK) cell, and macrophage (MP).

A pharmaceutical composition comprising a nucleic acid, vector or cell comprising a nucleotide sequence encoding the switchable CAR according to the invention and a pharmaceutically acceptable thinner or carrier.

Preferably, the pharmaceutical composition comprises cells comprising a nucleotide sequence encoding the switchable CAR according to the invention. In embodiments, the pharmaceutical composition further comprises a targeting module.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1•10⁵ to 1•10⁸ per mL of the nucleic acid, vector or cell comprising a nucleotide sequence encoding the switchable CAR according to the invention, and optionally, a concentration in the range of 1 ng/ml to 500 mg/ml of the targeting module.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferablyin a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the targeting module in a dosage quantity in the range of 25 µg/day to 100 mg/day, preferably dosage quantities in the range of 0.1 mg/day to 20 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In embodiments, the pharmaceutical composition comprises at least one furthertargeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, espedally preferred EphA1-10, EphA5 or EphB1-6; prostate specific antigens, preferably PSCA and PSMA; embryonic antigens, preferably CEA and fetal acetylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, disialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

A further aspect of the invention is a kit comprising
a) a nucleic acid, vector or cell comprising a nucleotide sequence encoding the switchable CAR according to one of the claims 1 to 10, and
b) a targeting module comprising a target cell-binding domain capable of binding a target antigen and a tag or tag-binding domain,
wherein the tag-binding domain of the targeting module binds to the tag of the switchable chimeric antigen receptor or the tag of the targeting module binds to the tag-binding domain of the switchable chimeric antigen receptor.

In embodiments, the targeting module is isolated. As used herein, the term "isolated" means altered or removed from the natural state. In embodiments, the targeting module is expressed as a recombinant protein. In further embodiments, the targeting module is chemically synthesized.

In embodiments, the targeting module is in monomeric, dimeric or polymeric form, preferably in monomeric form.

In further embodiments, the targeting module is monovalent, bivalent or multivalent.

In embodiments, the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, disialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands.

As used herein, the term "analogues" refers to molecules having a high degree of structural identity to the named antibodies, antibody fragments, proteins, peptides or low molecular weight organic ligands, preferably at least one atom, group of atoms, functional group or substructure is replaced with another group of atoms, e.g. a hydroxy group. In embodiments, an analogue of somatostatin (SRIF14) is octreotide or pasireotide. Advantageously, the analogues bind the identical antigens as the named antibodies, antibody fragments, proteins, peptides or low molecular weight organic ligands.

In embodiments, the analogues of the named antibodies, antibody fragments, proteins or peptides comprise modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, srylike high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In further embodiments, the target cell-binding domain is a soluble T cell receptor consisting of the alpha and beta or the gamma and delta chain of a T cell receptor (TCR).

In embodiments, the target cell-binding domain of the targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, disialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans.

In preferred embodiments, the at least one target cell-binding domain is an antibody, antibody fragment, a protein, a peptide or a low molecular weight organic ligand that binds to CD123.

In embodiments, the variable regions of the at least one target cell-binding domain, preferably the CD123-binding domain; comprises a humanized amino acid sequence.

In more preferred embodiments, the at least one target cell-binding domain is an antibody fragment that binds to CD123.

In some embodiments, the targeting module according to the invention is bivalent or multivalent and comprises at least one CD123-binding domain.

In embodiments, the different domains of the targeting module are linked with each other by a linker. The linker comprises a short sequence of preferably 20 to 30 amino acid residues. In embodiments, the targeting module comprises a flexible peptide sequence that is selected such that the domains have a three-dimensional folding that allows them to exhibit the specificity for effector cell and target cell binding.

Preferred linkers are glycine-serine linkers with the structure (GₓS_{y}) with x and y selected from 1 to 10, preferably 1 to 5. Mostly preferred are 2 to 5 repeats of the sequence G₄S₁ (SEQ ID No. 13). Moreover, linkers are preferred that are constituted of a peptide sequence that can increase the protease resistance of the antibody derivatives.

In embodiments, the linker of the tag-binding domain comprises 20 to 30 amino acids, preferably 25 amino acids. In embodiments, the linker has a sequence according to SEQ ID No. 14 or SEQ ID No. 15.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In preferred embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA or FcRn-binding peptides.

In embodiments, the length of the targeting module is in the range of 20 to 1600 amino acids, preferably 200 to 1300 amino acids.

In embodiments, the targeting module comprises one of the sequences according to SEQ ID No. 46 to SEQ ID No. 59.

In preferred embodiments, the targeting module comprises one of the sequences according to SEQ ID No. 48, SEQ ID No. 49, SEQ ID No. 51, SEQ ID No. 52, SEQ ID No. 55, SEQ ID No. 57, SEQ ID No. 58 or SEQ ID No. 59. More preferably, the targeting module comprises one of the sequences according to SEQ ID No. 51 or SEQ ID No. 58. Mostly preferred, the targeting module has one of the sequences according to SEQ ID No. 51 or SEQ ID No. 58.

In embodiments, the nucleic acid, vector or cell comprising a nucleotide sequence encoding a switchable chimeric antigen receptor and/or the targeting module are in the form of a pharmaceutical composition.

In embodiments, the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD133, CD135, CD150 CD181, CD182, CD184, CD223, CD229, CD269, CD273, CD274, CD276, CD279, CD319, CD366 and CD371, cytokine receptors, preferably interleukin receptors, especially preferred IL-8Rα, IL-8Rβ, IL-11Rα, IL-11Rβ, IL13Rα1; CXCR4, c-Met, mesothelin, members of the epidermal growth factor receptor family and mutants thereof, especially preferred ErbB1, ErbB2, ErbB3, ErbB4 or mutants thereof; members of the tumor necrosis factor receptor superfamily, claudins, ephrins, ephrin receptors, especially preferred EphA1-10, EphA5 or EphB1-6; fucosyl transferases, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, disialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands,
wherein the targeting module and the at least one further targeting module comprise different target cell-binding domains, and identical tag-binding domains or a tags.

In preferred embodiments, the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain of the further targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, disialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans,
wherein the targeting module and the at least one further targeting module comprise different target cell-binding domains, and identical tag-binding domains or tags.

In further embodiments, the at least one further targeting module comprises two target cell-binding domains binding to surface antigens selected from PSCA and PSMA, ErbB-1 and ErbB-2, PSCA and ErbB-2, PSMA and CEA, IL13Rα2 and ErbB-2, CD38 and CD269, CD19 and CD20, mesothelin and mucin 16, PD-L1 and ErbB-2.

In embodiments, the kit comprises one to three targeting modules, preferably one targeting module binding to CD123 and one or two further targeting modules.

In alternative embodiments, the kit comprises a nucleic acid, vector or cell encoding the targeting module. The nucleic acid, vector and/or cell are isolated.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

In embodiments, the switchable chimeric antigen receptor according to the invention, the nucleic acid, vector or cell according to the invention, the pharmaceutical composition according to the invention or the kit according to the invention are used as a medicament.

In embodiments, the switchable chimeric antigen receptor according to the invention, the nucleic acid, vector or cell according to the invention, the pharmaceutical composition according to the invention or the kit according to the invention are used for preparing a medication for therapeutic and/or diagnostic use in case of cancer, an infection or an autoimmune disease.

The term "autoimmune disorder" refers to an abnormal immune response of the body against substances and tissues normally present in the body (autoimmunity).

In embodiments, the switchable chimeric antigen receptor according to the invention, the nucleic acid, vector or cell according to the invention, the pharmaceutical composition according to the invention or the kit according to the invention are used in the treatment of cancer, infectious disease or autoimmune disease.

In embodiments, the switchable chimeric antigen receptor according to the invention, the nucleic acid, vector or cell according to the invention, the pharmaceutical composition according to the invention or the kit according to the invention are used for treating CD123-expressing tumors, preferably acute myeloid leukemia.

In embodiments, the switchable chimeric antigen receptor according to the invention, the nucleic acid, vector or cell according to the invention, are administered in combination with the targeting module.

As used herein, the term "administered in combination" refers to a treatment, wherein the targeting module is administered prior to, simultaneously with and/or after the administration of the nucleic acid, vector or cell comprising a nucleotide sequence encoding a switchable chimeric antigen receptor.

In embodiments, the targeting module is administered one hour to 2 days, more preferably 4 to 24 hours, prior to the administration of the nucleic acid, vector or cell comprising a nucleotide sequence encoding a switchable CAR. Advantageously, the administration of the targeting module prior to the administration of the nucleic acid, vector or cell comprising a nucleotide sequence encoding a switchable chimeric antigen receptor stimulates the switchable chimeric antigen receptors and increases the expansion of the switchable chimeric antigen receptor carrying effector cells and their accumulation at the target site.

In further embodiments, the targeting module is administered simultaneously with the nucleic acid, vector or cell comprising a nucleotide sequence encoding a switchable CAR.

In further embodiments, the targeting module is administered until, preferably in the range of 3 days to 30 days, after the administration of the nucleic acid, vector or cell comprising a nucleotide sequence encoding a switchable CAR. Furthermore, additional such doses of the targeting module may be administered following resting periods to reactivate the switchable CAR-carrying effector cells.

A further aspect of the invention is a method for stimulating a CAR-mediated immune response in a mammal, preferably a human, having cancer, an infectious or an autoimmune disease by administration of a nucleic acid, vector or cell comprising a nucleotide sequence encoding a switchable CAR and a targeting module, a pharmaceutical composition or a kit according to the invention, preferably to a subject in need thereof.

For therapeutic applications, a sterile pharmaceutical composition according to the invention or a sterile kit according to the invention, comprising a pharmacologically effective quantity of the nucleic acid, vector or cell comprising a nucleotide sequence encoding a switchable CAR according to the invention and a targeting module, is administered to a subject in order to treat the aforementioned illnesses.

In some embodiments, the method for stimulating a CAR-mediated immune response in a mammal; preferably, the method for treatment of cancer, infectious or autoimmune disease, comprises the following steps:
a) administering to a mammal an effective amount of a targeting module and
b) administering to the mammal an effective amount of a nucleic acid, vector or cell comprising a nucleotide sequence encoding a switchable CAR according to the invention,
   wherein the tag-binding domain of the targeting module binds to the tag of the switchable CAR or the tag of the targeting module binds to the tag-binding domain of the switchable CAR,
wherein the targeting module is administered to a mammal prior to, concurrent with or after the administration of the nucleic acid, vector or cell.

In preferred embodiments, the targeting module is administered on its own, preferably one hour to 2 days, more preferably 4 to 24 hours, prior to the administration of the nucleic acid, vector or cell and the targeting module is administered until, preferably in the range of 3 days to 30 days, after the administration of the nucleic acid, vector or cell. Furthermore, additional such doses of the targeting module may be administered following resting periods to reactivate the switchable CAR-carrying effector cells.

The invention is not limited to the embodiments shown and described, but also includes all embodiments having the same effect within the meaning of the invention. Furthermore, the invention is also not limited to the specifically described combinations of features, but may also be defined by any other combination of specific features of all the individual features disclosed as a whole, provided that the individual features are not mutually exclusive, or a specific combination of individual features is not explicitly excluded.

The following detailed description of exemplary embodiments of the invention is presented to enable any person skilled in the art to make and use the disclosed subject matter in the context of one or more particular implementations. Various modifications to the disclosed implementations will be readily apparent to those skilled in the art, and the general principles defined herein may be applied to other implementations and applications without departing from scope of the disclosure. Thus, the present disclosure is not intended to be limited to the described or illustrated implementations, but is to be accorded the widest scope consistent with the principles and features disclosed herein.
**Fig. 1** shows a schema of a switchable chimeric antigen receptor (CAR) with three domains, wherein the first domain is a tag-binding domain or a tag (exemplified as scFv), the second domain is an extracellular hinge and a transmembrane domain, and the third domain is a signal transduction domain, and the optional fourth domain is a short peptide linker in the extracellular portion of the receptor.

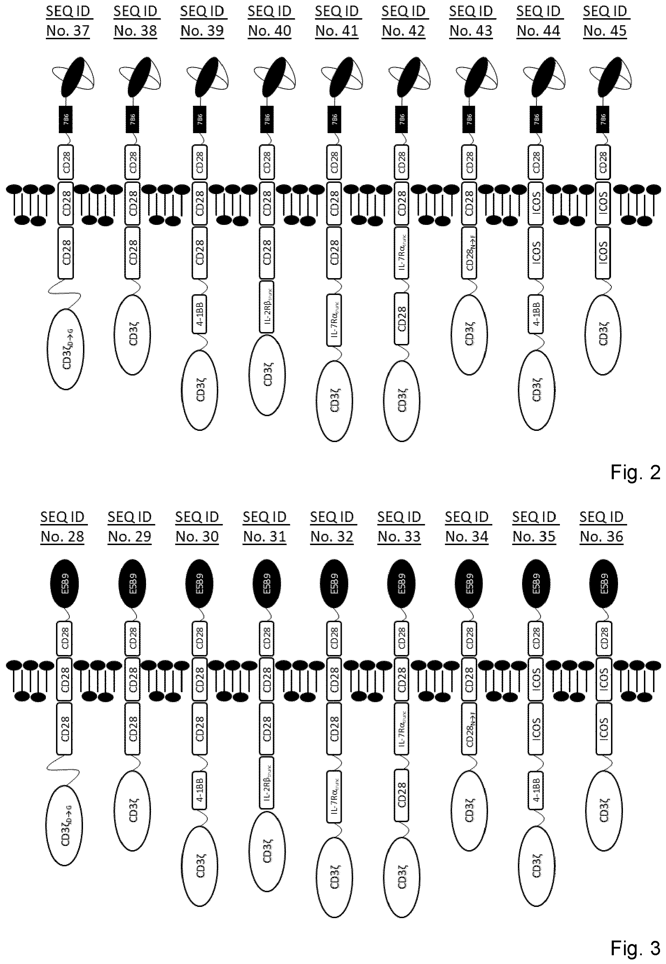

**Fig. 2** shows a schema of the structures of UniCAR variants according to the invention.
**Fig. 3** shows a schema of the structures of RevCAR variants according to the invention.

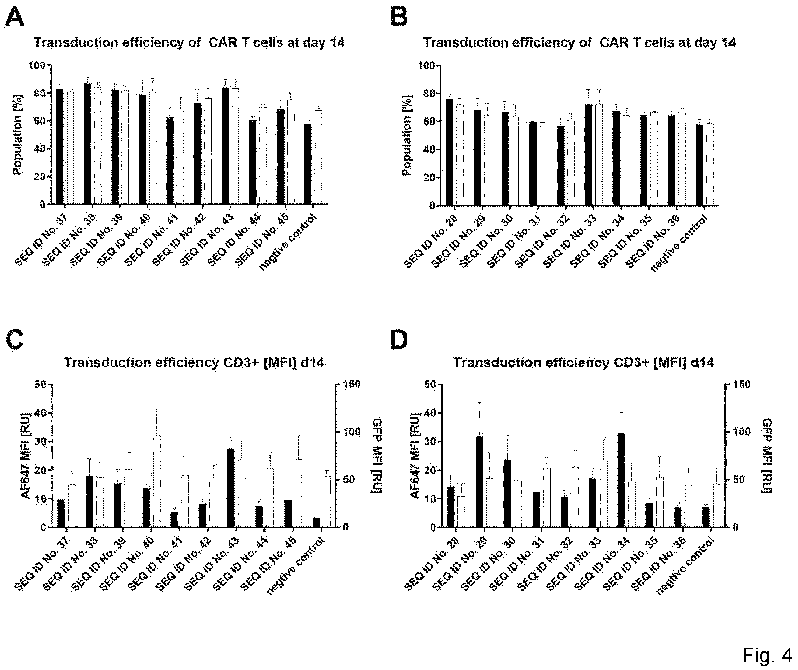

**Fig. 4** shows the transduction efficiency of UniCAR and RevCAR T cells on day 14 of the manufacturing process. Primary human T cells were isolated from peripheral blood mononuclear cells and transduced using lentiviral vectors containing UniCARs **(A, C)** or RevCARs **(B, D).** Transduction efficiency is analyzed by flow cytometric CAR staining and EGFP expression on day 14. Transduction efficiency **(A, B)** is assessed as percentage positive cells for CAR staining or EGFP expression. CAR Expression **(C, D)** is assessed as the mean fluorescence intensity (MFI) of the CAR or EGFP signal. Data is shown as mean ± SEM for at least four independent donors. Replicate experiments are shown as symbols.

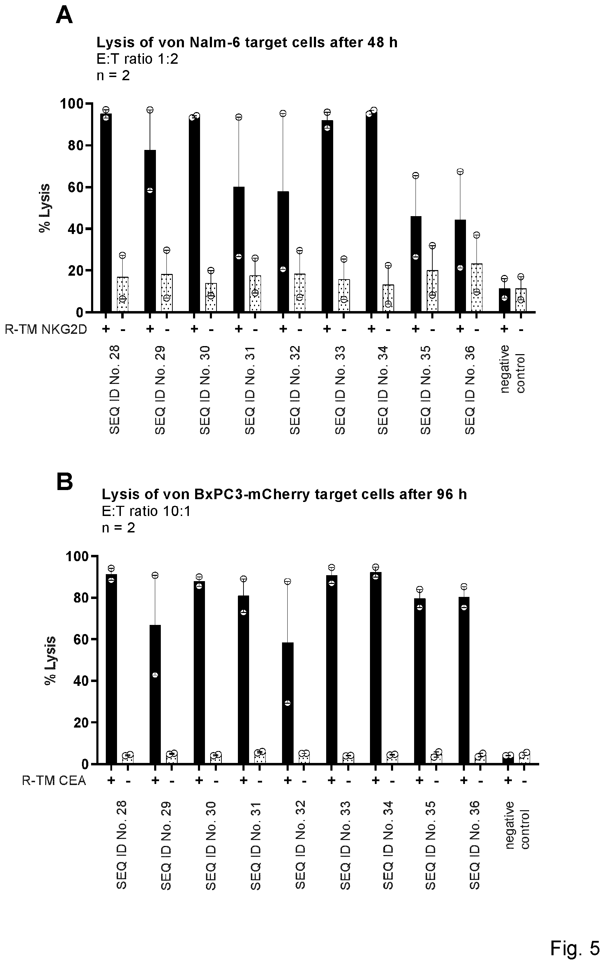

**Fig. 5** shows results of a cellular short-term cytotoxicity assay of RevCAR variants on **A)** Nalm-6 and **B)** BxPC3-mCherry target cells. T cell cytotoxic potential was assessed by co-incubating freshly thawed T cells with either Nalm-6 (A) or BxPC3-mCherry (B) target cells in presence of 50 nM targeting module. Data is presented as mean ± SEM of two independent donors with each donor shown as symbols. A) T cells were co-incubated with Nalm-6 target cells for 48 h at an E:T ratio of 1:2. Target cell lysis was calculated by normalizing the target cell count of each sample to a control sample where only target cells were present. Cell counts were assessed by flow cytometry B. T cells were co-incubated with BxPC3-mCherry target cells for 96 h at an E:T ratio of 10:1. Target cell lysis was assessed using the Incucyte^{®} Zoom by quantifying both living and dead target cells and calculating the proportion of dead target cells in relation to total target cells.

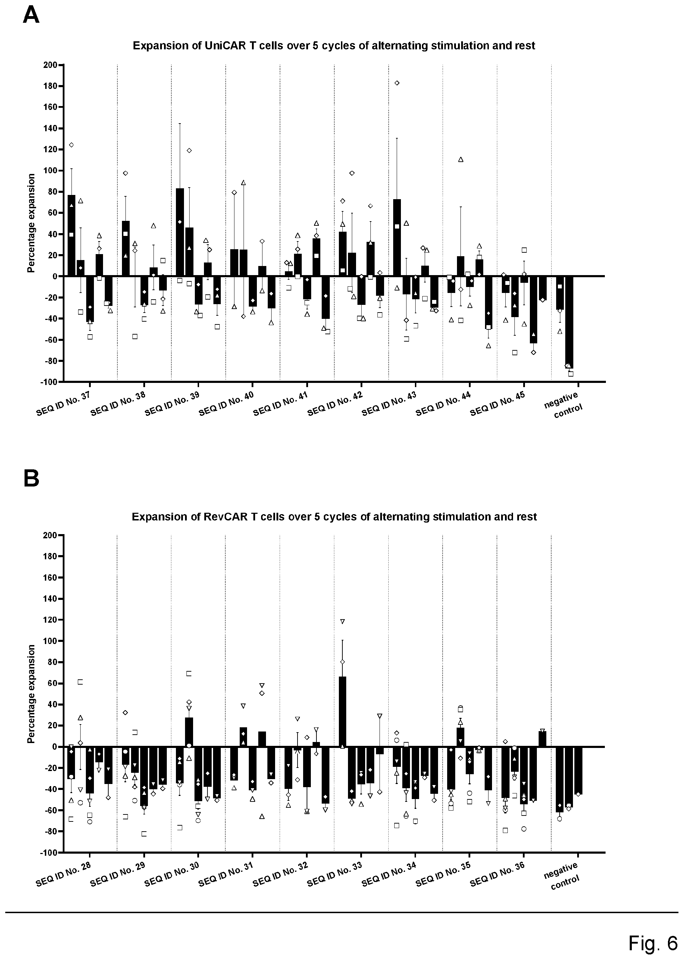

**Fig. 6** shows experimental results addressing the expansion of CAR T cells during a long-term re-challenge assay. CAR T cells were thawed and pre-cultured for 8 days before co-incubation with BxPC3-mCherry target cells for four days in presence of 10 nM targeting module against carcinoembryogenic antigen (CEA). At the end of four days (end of cycle 1 (eoc1)), T cells were harvested and characterized using flow cytometry and used for a short-term cytotoxicity assay. Remaining T cells were seeded without target cells for three days (cycle 2). This process was repeated for in total 18 days. UniCAR (A) and RevCAR (B) T cells were quantified and percentage expansion was calculated in relation to the T cell count seeded at the start of the cycle. Data is presented as mean ± SEM of at least three independent donors and experiment.

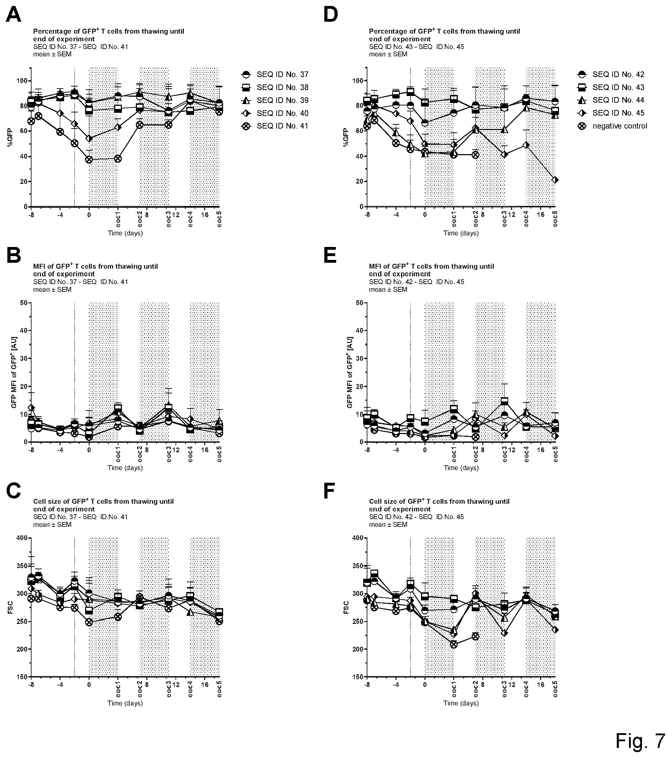

**Fig. 7** shows experimental results addressing longitudinal analysis of CAR expression of UniCAR T cells during a long-term re-challenge assay. The re-challenge assay was performed as described above. T cells were harvested and analyzed after each cycle for CAR expression and activation status indicated by cell size. The vertical line represents the start of the experiment. The dotted areas represent cycles where T cells and target cells were co-incubated. Non-dotted areas represent rest phases. SEQ ID No. 37 to 41 are shown in diagram **A, B** and **C.** SEQ ID No. 42 to 45 are shown in diagram **D, E** and **F.** The percentage of transduced cells over time is presented in diagram **A** and **D.** The CAR expression was assessed using the mean fluorescence intensity (MFI) and is shown in **B** and **E**. Cell size as an indicator for T cell activation is shown in **C** and **F.** Data is represented as mean ± SEM of at least three independent donors.

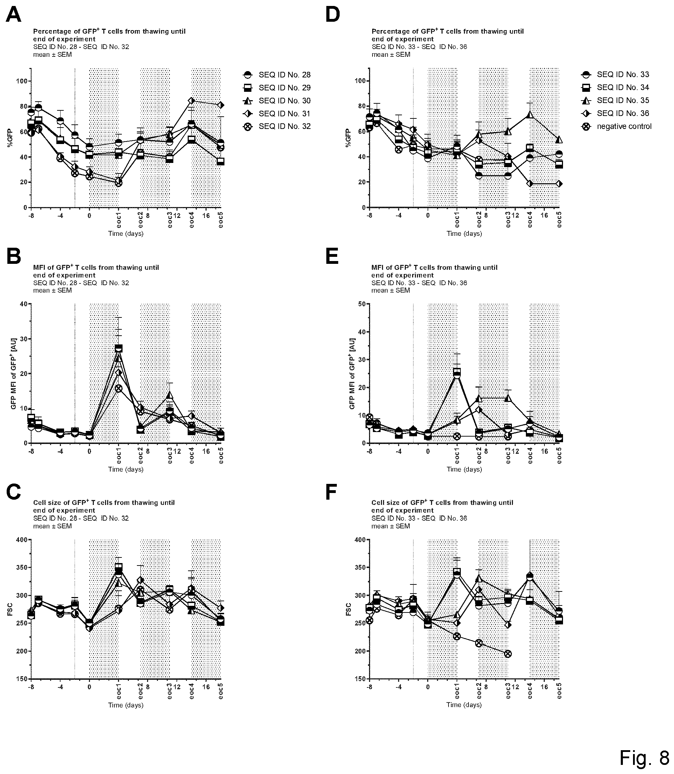

**Fig. 8** shows experimental results addressing longitudinal analysis of CAR expression of RevCAR T cells during a long-term re-challenge assay. The re-challenge assay was performed as described above. T cells were harvested and analyzed after each cycle for CAR expression and activation status indicated by cell size. The vertical line represents the start of the experiment. The dotted areas represent cycles where T cells and target cells were co-incubated. Non-dotted areas represent rest phases. SEQ ID No. 28 to 32 are shown in diagram **A**, **B** and **C**. SEQ ID No. 33 to 36 are shown in diagram **D, E** and **F**. The percentage of transduced cells over time is presented in diagram **A** and **D**. The CAR expression was assessed using the mean fluorescence intensity (MFI) and is shown in **B** and **E**. Cell size as an indicator for T cell activation is shown in **C** and **F.** Data is represented as mean ± SEM of at least three independent donors.

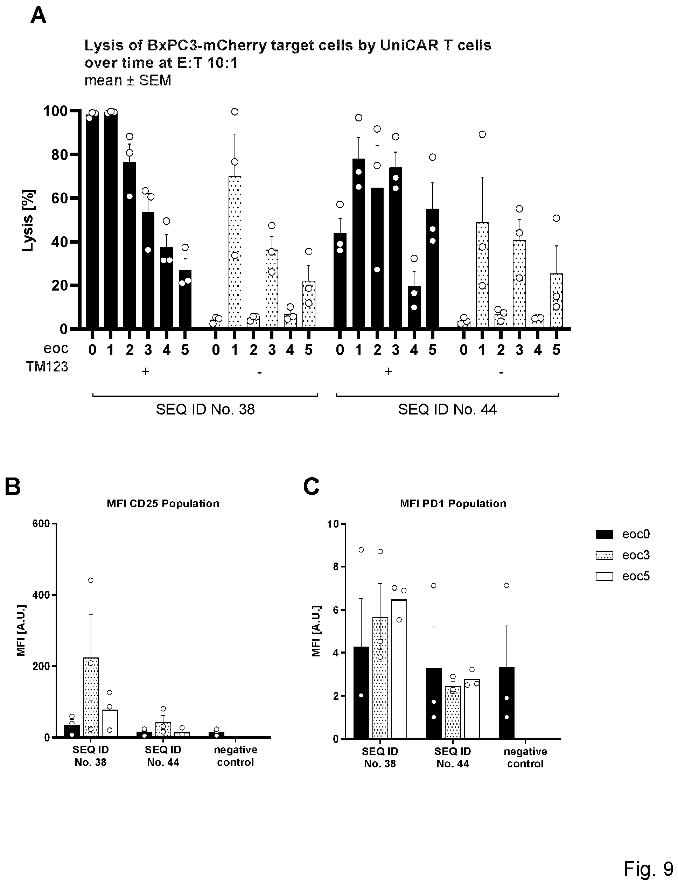

**Fig. 9** shows experimental results addressing long-term persistence of selected switchable UniCAR variants using a cell-based re-challenge assay. The re-challenge assay as performed as described above. Data is shown as mean ± SEM of at least three independent donors and experiments. Replicate experiments are shown as symbols. Pre-stimulated T cells were co-incubated with fresh BxPC3-mCherry target cells for 120 h either in presence of 10 nM targeting module (TM-CEA) (+) or without TM (-) at an E:T ratio of 10:1. **A)** Target cell lysis was assessed using the Incucyte^{®} Zoom by quantifying both living and dead target cells and calculating the proportion of dead target cells in relation to total target cells. **B/C)** At the end of co-incubation cycles T cells were characterized by flow cytometry for activation marker CD25 **(B)** and exhaustion marker **(C).** Shown is the mean fluorescence intensity (MFI) of the signal at the beginning of the experiment (eoc0), at the end of the second co-incubation cycle (eoc3) and at the end of the experiment (eoc5).

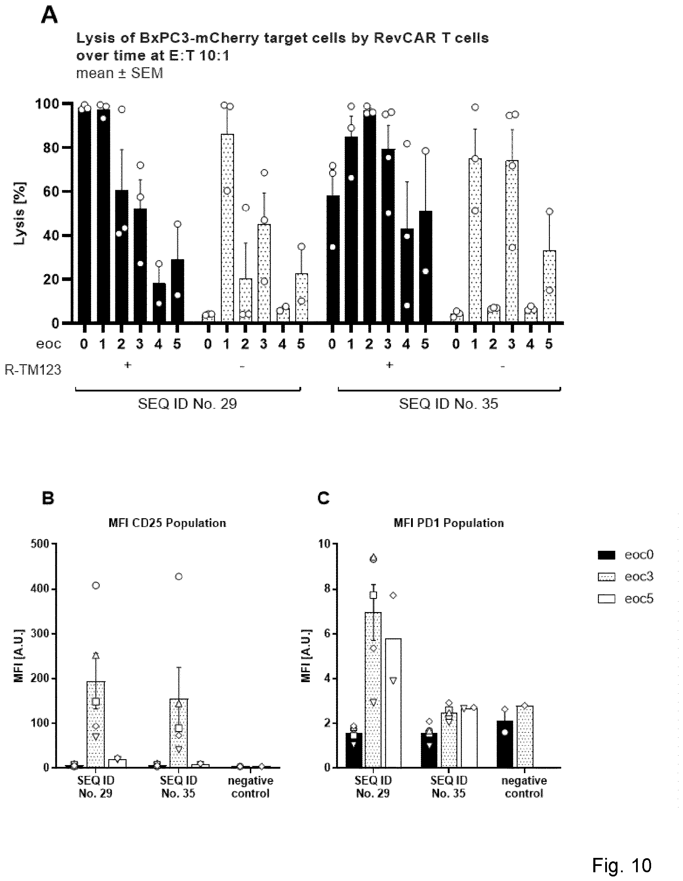

**Fig. 10** shows experimental results addressing long-term persistence of selected RevCAR variants using a cell-based re-challenge assay. The re-challenge assay as performed as described above. Data is shown as mean ± SEM of at least three independent donors and experiments. Replicate experiments are shown as symbols. Pre-stimulated T cells were co-incubated with fresh BxPC3-mCherry target cells for 120 h either in presence of 10 nM TM-CEA (+) or without TM (-) at an E:T ratio of 10:1. **A)** Target cell lysis was assessed using the Incucyte^{®} Zoom by quantifying both living and dead target cells and calculating the proportion of dead target cells in relation to total target cells. **B/C)** At the end of co-incubation cycles T cells were characterized by flow cytometry for activation marker CD25 **(B)** and exhaustion marker (C). Shown is the mean fluorescence intensity (MFI) of the signal at the beginning of the experiment (eoc0), at the end of the second co-incubation cycle (eoc3) and at the end of the experiment (eoc5).

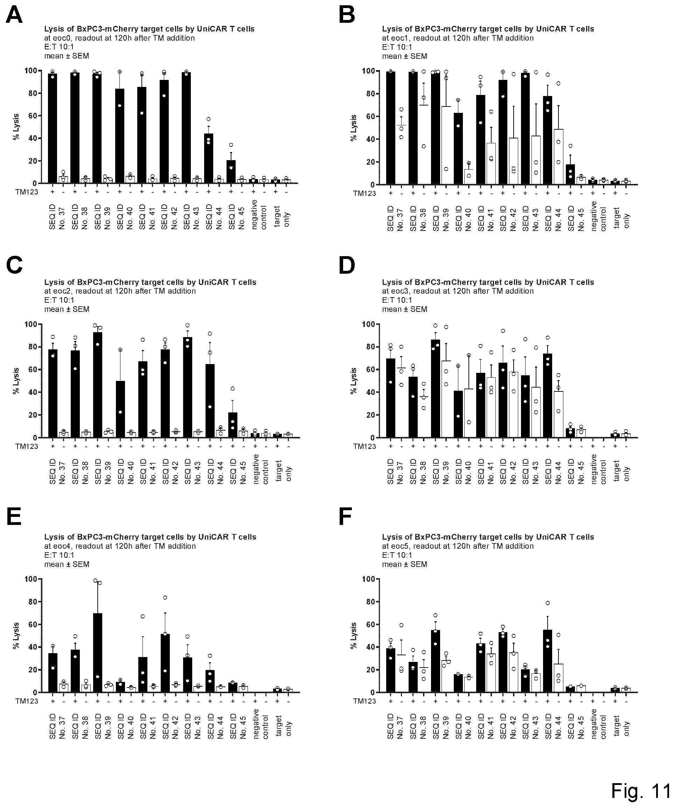

**Fig. 11** shows experimental results addressing the assessment of long-term cytotoxic potential during the re-challenge assay for UniCAR constructs over time. Pre-stimulated T cells were co-incubated with fresh BxPC3-mCherry target cells for 120 h either in presence of 10 nM TM-CEA (+) or without TM (-) at an E:T ratio of 10:1. Target cell lysis was assessed using the Incucyte^{®} Zoom by quantifying both living and dead target cells and calculating the proportion of dead target cells in relation to total target cells. Data is shown as mean ± SEM of at least three independent donors and experiments. Replicate experiments are shown as symbols. Each diagram represents data of one separate cycle: eoc0 **(A),** eoc1 **(B),** eoc2 **(C),** eoc3 **(D),** eoc4 **(E)** and eoc5 **(F).**

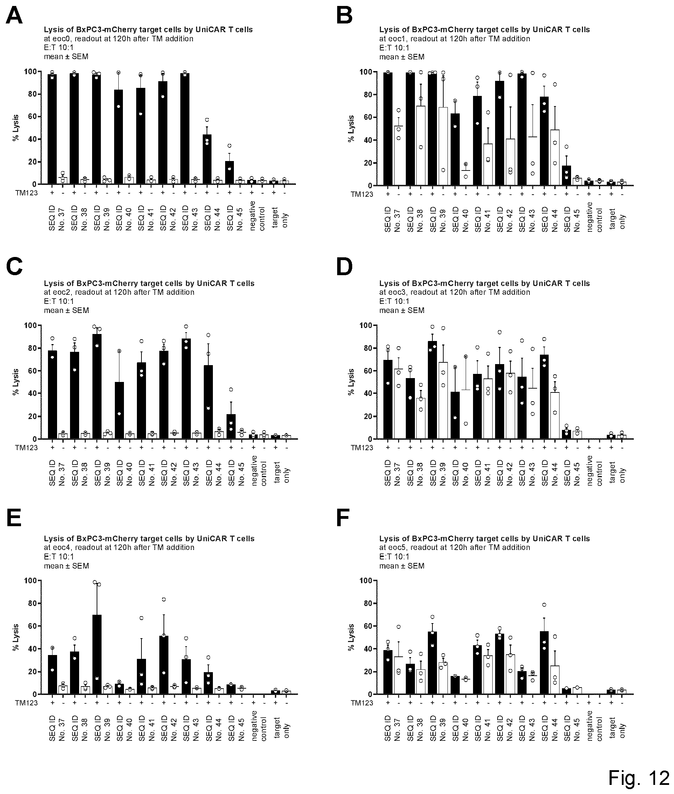

**Fig. 12** shows experimental results addressing the assessment of long-term cytotoxic potential during the re-challenge assay for RevCAR constructs over time. Pre-stimulated T cells were co-incubated with fresh BxPC3-mCherry target cells for 120 h either in presence of 10 nM TM-CEA (+) or without TM (-) at an E:T ratio of 10:1. Target cell lysis was assessed using the Incucyte^{®} Zoom by quantifying both living and dead target cells and calculating the proportion of dead target cells in relation to total target cells. Data is shown as mean ± SEM of at least three independent donors and experiments. Replicate experiments are shown as symbols. Each diagram represents data of one separate cycle: eoc0 **(A),** eoc1 **(B),** eoc2 **(C),** eoc3 **(D),** eoc4 **(E)** and eoc5 **(F).**

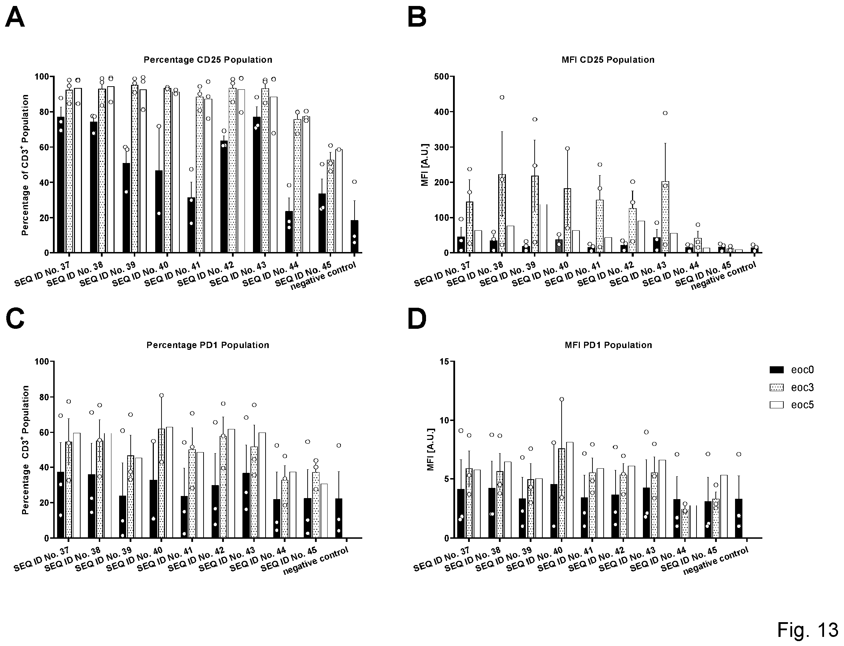

**Fig. 13** shows experimental results addressing the expression of activation and exhaustion markers of UniCAR variants during the long-term re-challenge assay. The re-challenge assay was performed as described above. At the beginning of the experiment (eoc0), after the second co-incubation (eoc3) and at the end of the experiment (eoc5) UniCAR T cells were analyzed by flow cytometry for the expression of activation marker CD25 **(A, B)** and exhaustion marker PD1 (C, D). Data is presented as percentage of the population expressing the marker **(A, C)** and the marker expression level per cell **(B, D)** measured by mean fluorescence intensity (MFI). Data is presented as mean ± SEM of at least three independent experiments and donors. Replicate experiments are shown as symbols.

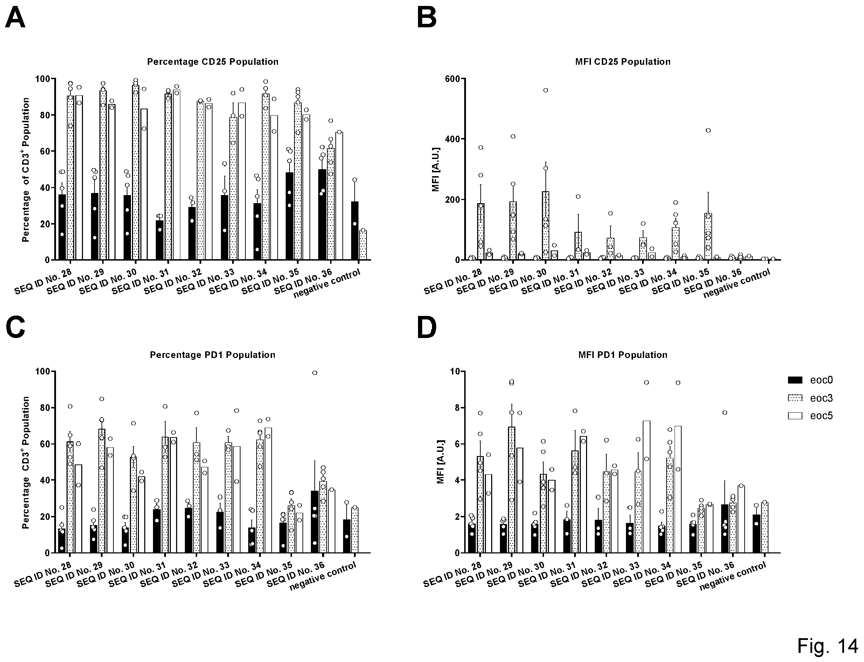

**Fig. 14** shows experimental results addressing the expression of activation and exhaustion markers of RevCAR variants during the long-term re-challenge assay. The re-challenge assay was performed as described above. At the beginning of the experiment (eoc0), after the second co-incubation (eoc3) and at the end of the experiment (eoc5) RevCAR T cells were analyzed by flow cytometry for the expression of activation marker CD25 **(A, B)** and exhaustion marker PD1 **(C, D).** Data is presented as percentage of the population expressing the marker **(A, C)** and the marker expression level per cell **(B, D)** measured by mean fluorescence intensity (MFI). Data is presented as mean ± SEM of at least three independent experiments and donors. Replicate experiments are shown as symbols.

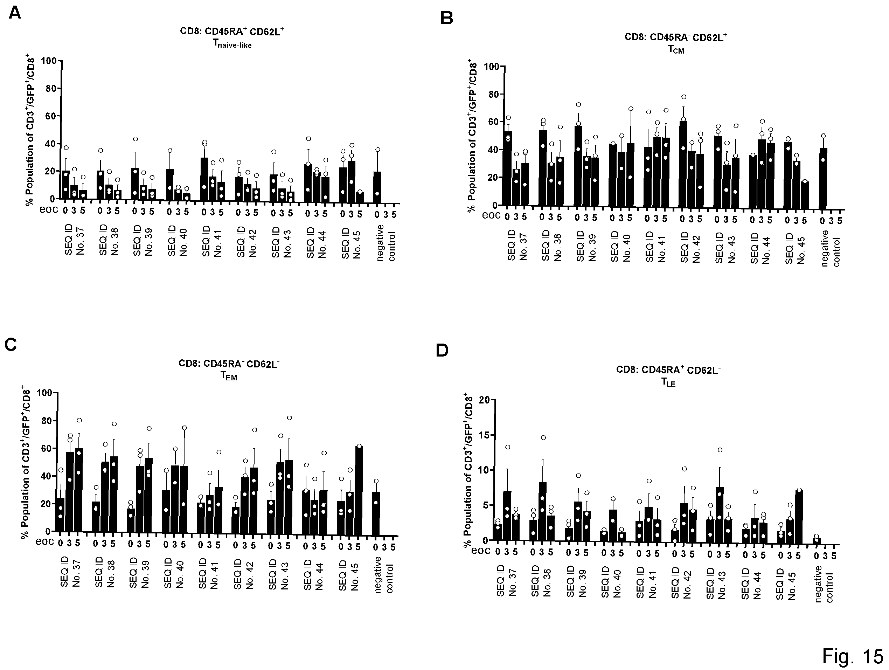

**Fig. 15** shows experimental results addressing the analysis of memory differentiation of UniCAR variants during the long-term re-challenge assay. The re-challenge assay was performed as described above. At the beginning of the experiment (eoc0), after the second co-incubation (eoc3) and at the end of the experiment (eoc5) UniCAR T cells were analyzed by flow cytometry for the expression of distinct markers to distinguish multiple memory phenotypes. Memory phenotype stages were distinguished using CD45Ra and CD62L expression. Memory phenotypes are defined as follows: **A)** naïve-like memory phenotype (CD45Ra+ CD62L+), **B)** central memory phenotype (T_{CM}: CD45Ra- CD62L+), C) effector memory phenotype (T_{EM}: CD45Ra- CD62L-) and **D)** late effector phenotype (T_{LE}: CD45Ra+ CD62L-). Data is only shown for CD8+ CAR T cells as mean ± SEM of at least three independent experiments and donors. Replicate experiments are shown as symbols.

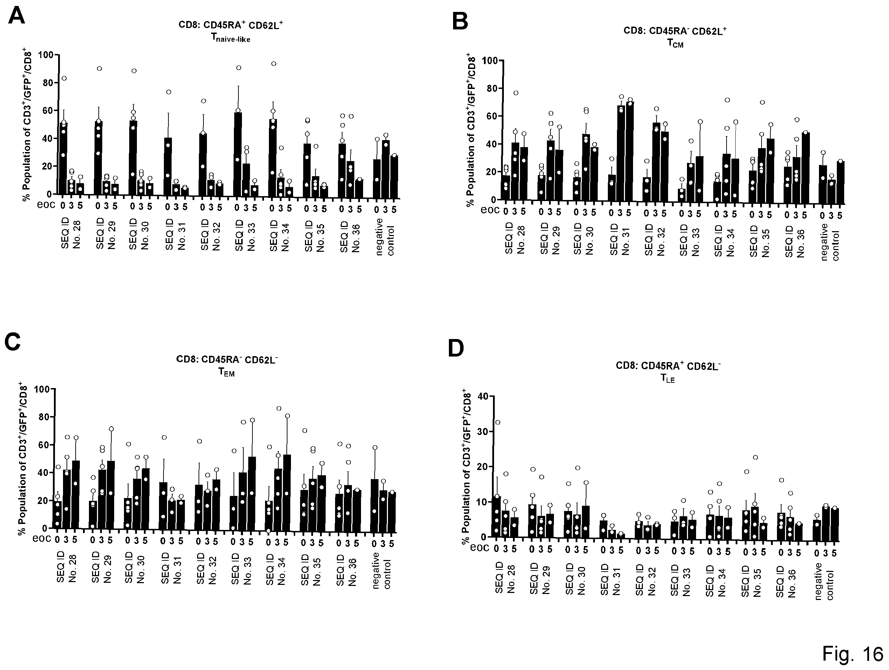

**Fig. 16** shows experimental results addressing the analysis of memory differentiation of RevCAR variants during the long-term re-challenge assay. The re-challenge assay was performed as described above. At the beginning of the experiment (eoc0), after the second co-incubation (eoc3) and at the end of the experiment (eoc5) RevCAR T cells were analyzed by flow cytometry for tie expression of distinct markers to distinguish multiple memory phenotypes. Memory phenotype stages were distinguished using CD45Ra and CD62L expression. Memory phenotypes are defined as follows: **A)** naïve-like memory phenotype (CD45Ra+ CD62L+), **B)** central memory phenotype (T_{CM}: CD45Ra- CD62L+), **C)** effector memory phenotype (T_{EM}: CD45Ra- CD62L-) and **D)** late effector phenotype (T_{LE}: CD45Ra+ CD62L-). Data is only shown for CD8+ CAR T cells as mean ± SEM of at least three independent experiments and donors. Replicate experiments are shown as symbols.

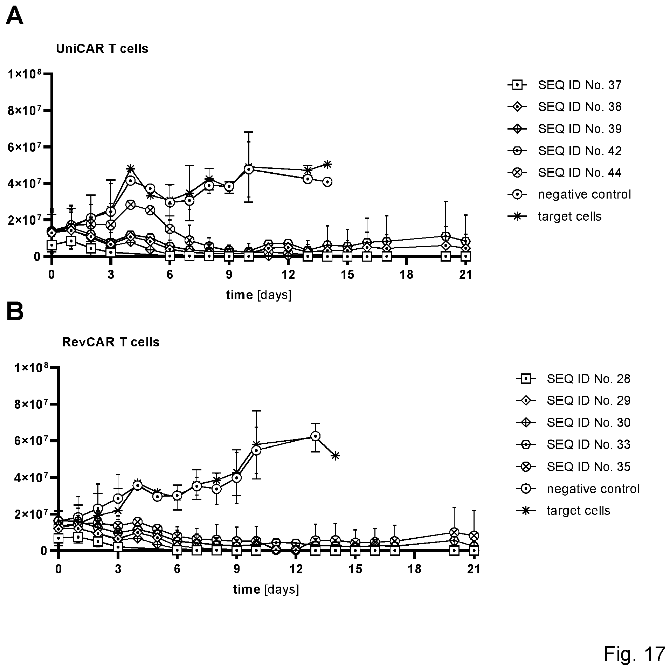

**Fig. 17** shows experimental results addressing long-term persistence of UniCAR **(A)** and RevCAR **(B)** variants using a 3D spheroid model. BxPC3-mCherry target cells were seeded in 2 % matrigel one day prior to the addition of thawed and precultured T cells. Co-incubation was performed at E:T 5:1 for 21 days in presence of 10 nM anti-CEA targeting module. Every 72 h or 96 h media and targeting module was refreshed. Spheroid intensity was observed using the Incucyte^{®} Zoom and data is presented as mean ± SD of three independent donors.

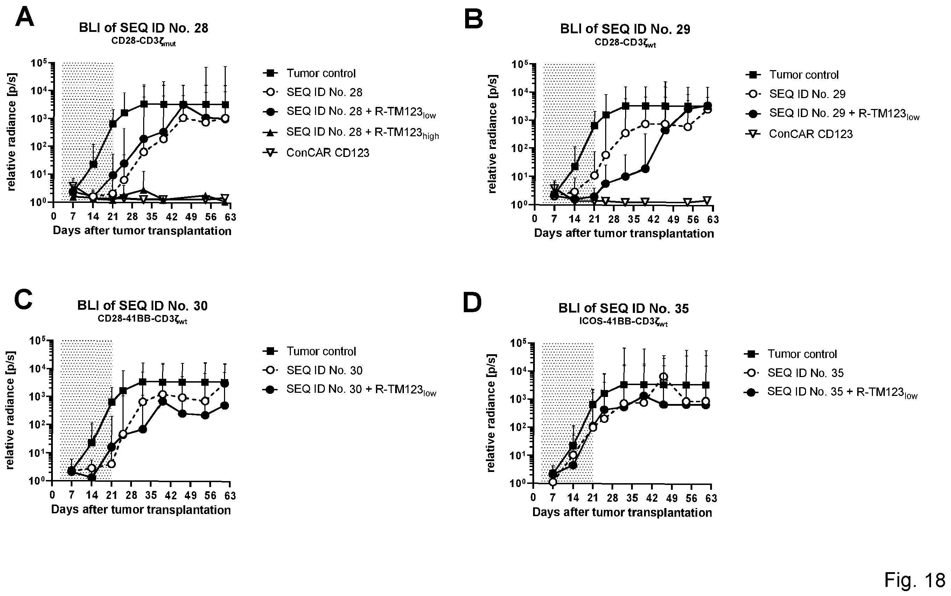

**Fig. 18** shows experimental results addressing *in vivo* anti-tumor efficacy of RevCAR variants in a leucemic xenograft mouse model. Three days prior to T cell transplantation, 1.0 × 10⁵ MV4-11 target cells were injected into the tail vein of NSG mice. MV4-11 target cells are transduced to express luciferase to enable longitudinal analysis of tumor progression by analysing the D-Luciferin fluorescence signal using the *in vivo* imaging system IVIS^{®} SpectrumCT. At the start of therapy, 5.0 × 10⁶ T cells were injected intravenously. Therapy groups were treated with anti-CD123 targeting module intraperitoneally (i.p.) twice daily at 1 µg/g or 3 µg/g mouse for three weeks on weekdays. As a positive control a non-switchable anti-CD123 CAR was used following the intracellular setup of SEQ ID No. 28 and SEQ ID No. 29. Tumor progression was observed for 63 days. Each data point represents the mean value of four biological replicates (four mice) with its corresponding statistical error of the mean. **A)** SEQ ID No. 28, **B)** SEQ ID No. 29, **C)** SEQ ID No. 30, **D)** SEQ ID No. 35.

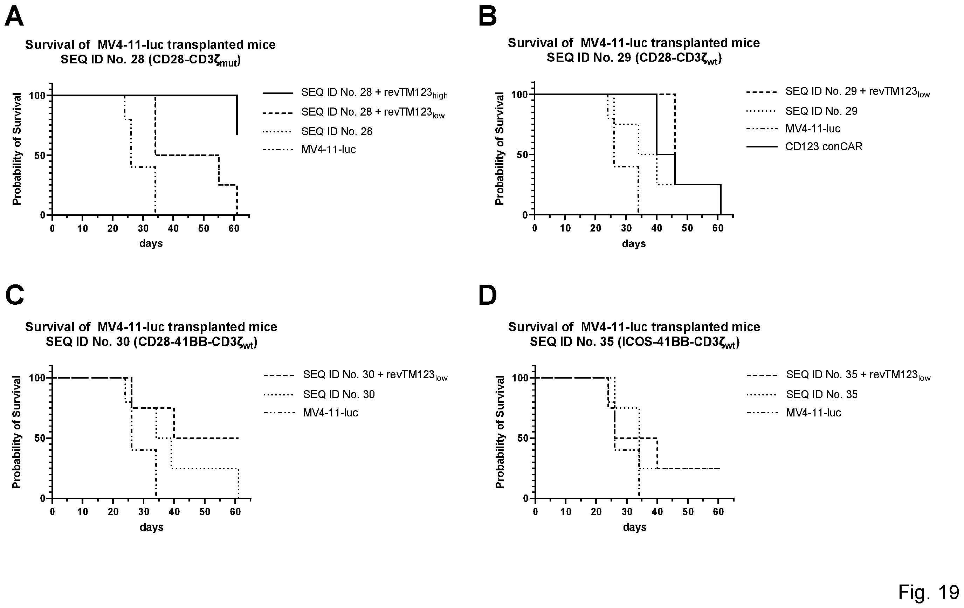

**Fig. 19** shows survival of NSG mice transplanted with luciferase expressing MV4-11 target cells and treated with RevCAR variants and anti-CD123 targeting module administration. Three days prior to T cell transplantation, 1.0 × 10⁵ MV4-11 target cells were injected into the tail vein of NSG mice. MV4-11 target cells are transduced to express luciferase to enable longitudinal analysis of tumor progression by analyzing the D-Luciferin fluorescence signal using the *in vivo* imaging system IVIS^{®} SpectrumCT. At the start of therapy, 5.0 × 10⁶ T cells were injected intravenously. Therapy groups were treated with anti-CD123 targeting module intraperitoneally (i.p.) twice daily at 1 µg/g or 3 µg/g mouse for three weeks on weekdays. As a positive control, a non-switchable anti-CD123 CAR was used following the intracellular setup of SEQ ID No. 28 and SEQ ID No. 29. Survival of NSG mice was observed for 63 days in total. Each data point represents the mean value of four biological replicates (four mice). **A)** SEQ ID No. 28, **B)** SEQ ID No. 29, **C)** SEQ ID No. 30, **D)** SEQ ID No. 35.

### Switchable CAR T cells according to the invention

The immune cells can be genetically engineered to express switchable CARs. For the genetical engineering to express UniCARs or RevCARs, a polynucleotide vector encoding the UniCAR or RevCAR and all necessary elements to ensure its expression in the genetically engineered immune cell is transferred into the immune cell. In particular, the UniCAR and RevCAR comprises IL-2LP (modified human IL-2 leader peptide), a tag-binding domain or tag, G₄S₁ (glycine-serine linker), ECD (extracellular domain), TMD (transmembrane domain) and at least two ICDs (intracellular domains) according to **Fig. 2** and **Fig. 3****.**

The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and cotransfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tagcontaining molecules for surface expression of switchable CARs or mabs directed against a fourth domain of switchable CARs from day 3 onwards after the final administration of virus supernatant. Switchable CAR transduced T cells can be propagated *in vitro* by culturing them under the supply of recombinant cytokines and activating anti-CD3 mabs.

In case the switchable CAR harbors the optional fourth domain, a peptide sequence forming a linear epitope for a mab, immune cells genetically modified to express switchable CARs can be specifically propagated *in vitro* by coating a mab or antibody fragments thereof binding to the fourth domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR-carrying immune cells and therefore enrichment of switchable CAR genetically modified immune cells in a mixed population.

The optional fourth domain, a peptide sequence forming a linear epitope for a mab, can be further utilized to enrich and purify switchable CAR-expressing immune cells from mixed populations. Enrichment and purification are performed with the help of a mab or antibody fragment thereof binding to the fourth switchable CAR domain to either mark switchable CAR-expressing cells for cell sorting or to transiently link the switchable CAR expressing immune cell to small particles, which can be utilized for cell isolation. In one aspect, switchable CAR-engrafted immune cells are incubated with the mab recognizing the fourth domain. Next, magnetic beads are added, which are conjugated with antibodies or fragments thereof directed against the species- and isotypespecific heavy and light chains of the mab binding to the optional fourth domain. Thus, switchable CAR-expressing immune cells and magnetic beads are linked and are trapped and separated from other immune cells in a magnetic field.

### Design of targeting modules

The targeting module TM123 is a soluble, recombinant fusion protein comprising two antibodyderived binding domains. One selectively binds to the target antigen CD123, the other recognizes the tag-binding domain or tag presented on the switchable CAR expressing cells (epitope E5B9 from the human La protein). Thus, TM123 functions as a bridging module between switchable CAR-T and a CD123-expressing target cancer cell (Fig. 1). The targeting module further comprises an 8x-histidine tag for detection and purification purposes at the C-terminus.

### In vitro characterization

Successful manufacturing of switchable CARs according to the invention was shown by both immunostaining of CARs using flow cytometry and EGFP expression **(****Fig. 4****).** All tested UniCAR and RevCAR constructs showed a transduction efficiency above 60 % with varying strength of CAR expression, measured via mean fluorescence intensity (MFI) on the cell surface.

### Cytotoxicity assay

The potency of the switchable CARs according to the invention and CD123-binding TMs to induce a tumor cell elimination was tested using a suspension cell based co-cultivation assay with Nalm-6 target cells (E:T 1:2) **(****Fig. 5A****)** and BxPC3 target cells (E:T 10:1) **(****Fig. 5B****)** in the presence of targeting module concentrations of 50 nM R-TM NKG2D (Nalm-6) or R-TM CEA (BxPC3). Switchable CAR T cells were incubated with the target cells at an E:T ratio of 1:2 for 48 h (Nalm-6) or 10:1 for 96 h (BxPC3). Target cells were quantified by flow cytometry or Incuyte^{®}Zoom. For flow cytometry based cytotoxicity lysis was calculated normalizing the cell count of each sample to a control sample where only tumor cells were plated. For Incucyte^{®} -based cytotoxicity both living and dead target cells were quantified lysis was assessed by calculating the proportion of dead target cells in relation to total target cells. Furthermore, as negative control constructs without any intracellular signaling domain were used (SEQ ID No. 60 or SEQ ID No. 61). All RevCAR constructs showed a cytotoxic potential in the range of 50 to 100%.

### Long-term efficacy

The long-term efficacy was tested by cycles of repeated stimulation of CAR T cells with fresh target cells for 4 days and alternating this stimulation with rest phases for 3 days. At the end of each cycle the T cells were harvested and characterized by flow cytometry regarding cell count CAR expression measured by EGFP and cell size. Results thereof are shown in **Fig. 6****,** **Fig. 7** and **Fig. 8****,** respectively. Additionally, a short-term cytotoxicity assay was set up, co-incubating the pre-stimulated CAR T cells and fresh BxPC3-mCherry target cells in presence of 10 nM anti-CEA targeting module (see above) **(****Fig. 11****,** **Fig. 12****).** Lastly, pre-stimulated CAR T cells were characterized in depth by flow cytometry regarding activation **(****Fig. 13A, Fig. 13B****,** **Fig. 14A, Fig 14B****),** exhaustion **(****Fig. 13C, Fig. 13D****,** **Fig. 14C, Fig 14D****)** and memory differentiation **(****Fig. 15****,** **Fig. 16****)** at three different time points during the long-term assay. The results of the cytotoxicity assay and the in-depth flow cytometry characterization of two selected switchable CARs according to the invention are shown in summery in **Fig. 9** and **Fig. 10** (RevCAR). All tested CAR variants show complex behavior when tested in long-term re-challenge assays. It becomes obvious that switchable CARs according to the invention, namely SEQ ID No. 28, SEQ ID No. 29, SEQ ID No. 30, SEQ ID No. 33, SEQ ID No. 35, SEQ ID No. 37, SEQ ID No. 38, SEQ ID No. 40 and SEQ ID No. 44 show sustained cytotoxic functionality after repeated stimulation with target cells. Switchable CARs according to the invention namely SEQ ID No. 35 and SEQ ID No. 44 display lower strength of activation leading to lower exhaustion of these CAR constructs at the end of the experiment.

Furthermore, the long-term efficacy was tested in 3D spheroid assays **(****Fig. 17****),** wherein switchable CAR T cells and 10 nM anti-CEA targeting module were incubated with spheroids of BxPC3-mCherry target cells seeded in 2 % matrigel at an E:T of 5:1. The spheroids were scanned using the Incucyte^{®} Zoom and CAR T cell count was determined after 7, 14 and 21 days. During the experiment, medium and targeting module was refreshed every 72 to 96 h. All tested switchable CARs control spheroid outgrow with some constructs even leading to complete spheroid eradication.

### In vivo characterization

The switchable CARs according to the invention were further characterized *in vivo* using mice, wherein 1 × 10⁵ MV4-11 luc cells were injection intravenously. Three days later, 5 × 106 RevCAR T cells were injection intravenously and targeting module administration was started. The therapy groups were treated with anti-CD123 targeting module administered intraperitoneally (IP) twice a day (1 µg/g or 3 µg/g mouse) for a total of three weeks on weekdays. Tumor progression was regularly checked one a week by fluorescence imaging using the IVIS^{®} SpectrumCT system. Mice were checked daily for signs of disease progression. If mice reached endpoint criteria, mice were sacrificed, and organs analyzed by flow cytometry. The results are shown in **Fig. 18** and **Fig. 19****.**

### Cited non-patent literature:

Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.

Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.

Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.

Reinke AW, Grant RA, Keating AE (2010) A Synthetic Coiled-Coil Interactome Provides Heterospecific Modules for Molecular Engineering. JACS 132, 6025-6031.

Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.

Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.

Weber EW, Parker KR, Sotillo E, Lynn RC, Anbunathan H, Lattin J, Good Z, Belk JA, Daniel B, Klysz D, Malipatlolla M, Xu P, Bashti M, Heitzeneder S, Labanieh L, Vandris P, Majzner RG, Qi Y, Sandor K, Chen LC, Prabhu S, Gentles AJ, Wandless TJ, Satpathy AT, Chang HY, Mackall CL (2021) Transient rest restores functionality in exhausted CAR-T cells through epigenetic remodeling. Science 372 (6537), eaba1786.

### List of reference signs

- 1: a tag-binding domain or tag
- 2: an extracellular hinge and a transmembrane domain
- 3: a signal transduction domain
- 4: a short peptide linker

## Claims

1. A switchable chimeric antigen receptor comprising
a. a tag or tag-binding domain,
b. an extracellular hinge and transmembrane domain comprising an extracellular hinge and a transmembrane domain of CD8α, CD28, ICOS (CD278) or mutants and combinations thereof,
c. an intracellular signaling domain that comprises at least two signal transduction domains independently selected from the group comprising a cytoplasmic region of CD3, CD28, 4-1BB (CD137), ICOS (CD278), IL-2, IL-7, IL-15 or IL-21 and mutants thereof.

2. The switchable chimeric antigen receptor according to claim 1, wherein the tag is a peptide epitope tag.

3. The switchable chimeric antigen receptor according to claim 2, wherein the peptide epitope tag is a myc-tag, a His-tag, a short linear peptide sequence from yeast transcription factor GCN4, a leucine zipper sequence or a short linear peptide sequence from a human protein.

4. The switchable chimeric antigen receptor according to one of the claims 1, wherein the tag-binding domain is an antibody or antigen-binding fragment, a protein or a peptide.

5. The switchable chimeric antigen receptor according to one of the claims 1 or 4, wherein the tag-binding domain is an antibody, antigen-binding fragment, a protein or a peptide binding to a myc-tag, a His-tag, a short linear peptide sequence from yeast transcription factor GCN4, a leucine zipper sequence or a short linear peptide sequence from a human protein.

6. The switchable chimeric antigen receptor according to one of the claims 1 to 5, wherein the intracellular signaling domain comprises two or three signal transduction domains selected from the group comprising CD28, 4-1BB, ICOS, CD3ζ, IL-7Rα and mutants thereof.

7. The switchable chimeric antigen receptor according to one of the claims 1 to 6, wherein the intracellular signaling domain comprises at least a cytoplasmic region of CD3ζ or mutants thereof.

8. The switchable chimeric antigen receptor according to one of the claims 1 to 7, wherein the switchable chimeric antigen receptor is a universal chimeric antigen receptor comprising a tag-binding domain.

9. The switchable chimeric antigen receptor according to one of the claims 1 to 7, wherein the switchable chimeric antigen receptor is a reversible chimeric antigen receptor comprising a tag.

10. The switchable chimeric antigen receptor according to one of the claims 1 to 9 comprising a sequence according to SEQ ID No. 28 to SEQ ID No. 45.

11. A nucleic acid, vector or cell comprising a nucleotide sequence encoding the switchable CAR according to one of the claims 1 to 10.

12. The vector according to claim 11, wherein the vector is selected from the group comprising a DNA vector, an RNA vector, a plasmid, a lentiviral vector, retroviral vector, adenoviral vector and an adeno-associated viral vector.

13. The vector of claim 12, further comprising a promoter, wherein the promoter is selected from the group comprising an EF-1 promoter, a CMV IE gene promoter, an EF-1α promoter, an ubiquitin C promoter, or a phosphoglycerate kinase (PGK) promoter.

14. The cell according to claim 11, wherein the cell is an immune effector cell selected from the group comprising a cytotoxic T lymphocyte, regulatory T cell, Natural Killer cell, and macrophage.

15. A pharmaceutical composition comprising a nucleic acid, vector or cell comprising a nucleotide sequence encoding the switchable CAR according to one of the claims 1 to 10 and a pharmaceutically acceptable thinner or carrier.

16. A kit comprising
a) a nucleic acid, vector or cell comprising a nucleotide sequence encoding the switchable CAR according to one of the claims 1 to 10, and
b) a targeting module comprising a target cell-binding domain capable of binding a target antigen and a tag or tag-binding domain,
wherein the tag-binding domain of the targeting module binds to the tag of the switchable chimeric antigen receptor or the tag of the targeting module binds to the tag-binding domain of the switchable chimeric antigen receptor.

17. The kit according to claim 16, wherein the target cell-binding domain of the targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans.

18. The kit according to claim 16 or 17, wherein the length of the targeting module is in the range of 20 to 1600 amino acids, preferably 200 to 1300 amino acids.

19. The kit according to one of the claims 16 to 18, wherein the nucleic acid, vector or cell comprising a nucleotide sequence encoding a reversible chimeric antigen receptor and/or the targeting module are in the form of a pharmaceutical composition.

20. The kit according to one of the claims 16 to 19, wherein the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain of the further targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6 CD52, CD66a, CD66b, CD66c, CD66d, CD66e, CD66f, CD90, CD99, CD123, CD133, CD135, CD150 CD181, CD182, CD184, CD223, CD229, CD269, CD273, CD274, CD276, CD279, CD319, CD366 and CD371, cytokine receptors, CXCR4, c-Met, mesothelin, members of the epidermal growth factor receptor family and mutants thereof, members of the tumor necrosis factor receptor superfamily, claudins, ephrins, ephrin receptors, fucosyl transferases, prostate specific antigens, embryonic antigens, members of the vascular endothelia growth factor family, EpCAM, AFP, members of the intercellular adhesion molecule family, C-type lectins, integrins, members of the mucin protein family, FSHR, HMW-MAA, FBP, folate receptors, somatostatin receptors, ligands of the NKG2D receptor, members of the epithelia glycoprotein family, diasialogangliosides, glypicans, G protein-coupled receptors, human papillomavirus proteins, cancer-testis antigens, fibroblast activation proteins, members of the carbonic anhydrase family, members of the carbohydrate antigen family, Notch ligands, MCSP, glycoprotein A33, guanylate cyclase 2C and tumor-specific glycans,
wherein the targeting module and the at least one further targeting module comprise different target cell-binding domains, and identical tag-binding domains or tags.

21. The switchable chimeric antigen receptor according to one of the claims 1 to 10, the nucleic acid, vector or cell according to one of the claims 11 to 14, the pharmaceutical composition according to claim 15 or the kit according to one of the claims 16 to 20 for use as a medicament.

22. The switchable chimeric antigen receptor according to one of the claims 1 to 10, the nucleic acid, vector or cell according to one of the claims 11 to 14, the pharmaceutical composition according to claim 15 or the kit according to one of the claims 16 to 20 for use in the treatment of cancer, infectious disease or autoimmune disease.
